# EUROPÄISCHE PATENTSCHRIFT

(11) **EP 4 215 128 B1**
(45) Veröffentlichungstag und Bekanntmachung des Hinweises auf die Patenterteilung: **18.06.2025**
(21) Anmeldenummer: 23155248.0
(22) Anmeldetag: 09.01.2020
(51) Int. Cl.: A61B 17/12, A61B 17/00

(54) **OKKLUDER, SYSTEM AUS OKKLUDER UND EINFÜHRKATHETER UND VERFAHREN ZUR BEREITSTELLUNG DES SYSTEMS**
OCCLUDER, OCCLUDER SYSTEM AND INSERTION CATHETER, AND METHOD FOR PROVIDING SYSTEM
DISPOSITIF D'OCCLUSION, SYSTÈME DE DISPOSITIF D'OCCLUSION ET CATHÉTER D'INTRODUCTION ET PROCÉDÉ DE FOURNITURE DU SYSTÈME

(30) Priorität: 10.01.2019 DE 102019100530
(43) Veröffentlichungstag der Anmeldung: 26.07.2023
(62) Teilanmeldung aus: 20701916.7
(73) Patentinhaber: Qatna Medical GmbH, 72379 Hechingen (DE)
(72) Erfinder: CENTOLA, Marcos, 72379 Hechingen-Stein (DE)
(74) Vertreter: DREISS Patentanwälte PartG mbB

(56) Entgegenhaltungen:
- WO-A1-2017/157316
- WO-A2-2006/036837
- DE-A1- 102015 104 785
- US-A1- 2011 184 439
- US-A1- 2017 239 036
- US-A1- 2018 338 832

## Beschreibung

Die Erfindung betrifft einen Okkluder zum Verschließen der auricula cordis sinistra eines Patienten. Die Erfindung betrifft auch ein dazugehöriges System zur Einführung eines Okkluders in einen Patienten sowie ein Verfahren zur Bereitstellung des Systems.

Die auriculae atrii oder Vorhofohren sind Ausstülpungen der Vorhöfe des Herzens bei Säugetieren. Das linke Vorhofohr, medizinisch als auricula cordis sinistra (englisch: Left Atrial Appendage (LAA)) bezeichnet, liegt neben dem Strang der Lungenarterie und ist, insbesondere bei Patienten mit Vorhofflimmern, ein häufiger Entstehungsort für Blutgerinnsel, die zu einem Schlaganfall führen können. Die Verhinderung von Thromben in der auricula cordis sinistra stellt daher eine wirksame Schlaganfallprophylaxe bei gefährdeten Patienten dar.

Für diese Schlaganfallprophylaxe wurden Implantate entwickelt, die in die Ausstülpungen eingesetzt werden und den Zugang beispielsweise über eine Teflonfolie verschließen. In der angelsächsischen Literatur werden diese Implantate als LAA (LAA: Left Atrial Appendage)-Okkluder bezeichnet. Diese Implantate werden in die Ausstülpungen eingesetzt und dort insbesondere mittels Verankerungselementen verankert, so dass sie insbesondere über ihren proximalen Endbereich den Zugang in die Ausstülpungen fluiddicht abschließen. Die Einbringung erfolgt meist über endovaskuläre Techniken, d.h. insbesondere mit einem Einführkatheter, durch den das Implantat an den Einsatzort verbracht wird. Dabei werden die Okkluder insbesondere in volumenreduzierter Form an den Einsatzort verbracht und dort expandiert. Für die Okkluder werden dabei in der Regel selbstexpandierende Materialien verwendet, beispielsweise Formgedächtnislegierungen. Ein derartiger Okkluder ist aus der WO 2015/079023 A1 vorbekannt.

Ferner offenbart die DE 10 2015 104 785 A1 eine Vorrichtung zum Verschließen der Ausstülpungen mittels eines Verschlusselements, wobei zwei Verankerungselemente das Verschlusselement ohne Verletzung von Gewebe fixieren.

Zudem sind aus der US 2018/0338832 A1 und der WO 2013/158608 A1 Vorrichtungen zum Anordnen unter anderem an der Mitralklappe mittels eines minimal-invasiven Eingriffs bekannt, bei der diese durch eine künstliche Klappe ersetzt wird oder dessen Funktion verbessert wird.

Außerdem offenbart die EP 1 729 682 B1 eine endoluminale Gefäßprothese, welche als Stent in die Aorta eingebracht wird und im expandierten Zustand ein Aneurysma (Aussackung) behebt.

Ferner offenbart die US 2011/184439 A1 einen Okkluder zum beidseitigen Verschließen eines Lochs in einer Gefäßwand, wobei der Okkluder zwei mit einem biologischen Material versehene Schirme und einen die beiden Schirme verbindenden zylindrischen Mittelabschnitt umfasst. Ein weiterer Okkluder zum Verschließen eines Lochs in einer Gefäßwand ist in der WO2006/036837A2 gezeigt.

Die WO 2017/157316 A1 offenbart einen Okkluder zum Verschließen der auricula cordis sinistra eines Patienten. Der Okkluder umfasst einen selbstexpandierbaren Rahmen. Im expandierten Zustand weist der Okkluder eine im Wesentlichen kugelförmige Außenkontur auf.

Der vorliegenden Erfindung liegt insbesondere die Aufgabe zugrunde, die Biokompatibilität der bekannten Okkluder zu verbessern.

Diese Aufgabe wird durch einen Okkluder mit den Merkmalen des Anspruchs 1 vorgesehen. Demnach wird ein Okkluder, insbesondere zum Verschließen der auricula cordis sinistra eines Patienten, vorgeschlagen. Der Okkluder umfasst dabei einen selbstexpandierbaren Rahmen, wobei an dem Rahmen ein diesen wenigstens abschnittsweise abdeckendes biologisches Gewebe angeordnet ist. Das biologische Gewebe ist insbesondere an einer Außenseite des Rahmens angeordnet. Die Verwendung von biologischem Gewebe dient insbesondere zur Erhöhung der Biokompatibilität des Okkluders. Dabei kann das biologische Gewebe insbesondere an der Außenseite des Rahmens angeordnet sein und somit in Kontakt mit dem natürlichen Gewebe des Patienten und/oder mit zirkulierendem Blut des Patienten sein. Insofern kann das biologische Gewebe insbesondere dazu dienen, im angeordneten Zustand des Okkluders die auricula cordis sinistra fluiddicht zu verschließen. Denkbar ist auch, dass nach der Anordnung des Okkluders in der auricula cordis sinistra eine natürliche Zell- oder Hautschicht auf dem biologischen Gewebe ausgebildet wird und somit das biologische Gewebe überwuchert. Insbesondere hierbei kann durch das Vorsehen des biologischen Gewebes eine erhöhte Biokompatibilität erzielt werden. Dabei kann insbesondere vorgesehen sein, dass das biologische Gewebe anstatt einer Teflonbespannung, wie in der WO 2015/079023 A1 offenbart, oder einer Bespannung aus einem sonstigen synthetischen Material verwendet wird. Dadurch kann insgesamt die Biokompatibilität des Okkluders verbessert werden.

Das biologische Gewebe ist dabei insbesondere durch Behandlung mittels einer Vernetzungsmethode stabilisiert. Das Vernetzen kann vorzugsweise mit dem cross-link-Verfahren unter Verwendung von insbesondere Glutaraldehyd und/oder Alkohol erfolgen. Das biologische Gewebe kann zudem zur Sterilisation und /oder Haltbarmachung mit einem flüssigen Medium dauerhaft benässt oder benässt und dann getrocknet sein. Denkbar ist, dass das biologische Gewebe zunächst mit einem flüssigen Medium sterilisiert und dann getrocknet, insbesondere vakuumgetrocknet wird. Das flüssige Medium ist vorzugsweise Glycerol und/oder ein einbis dreiwertiger Alkohol.

Der Rahmen kann insbesondere aus einem flexiblen, selbstexpandierenden Material bestehen oder ein solches Material umfassen. Dieses Material kann ein Metall oder ein Kunststoff sein. Vorteilhafterweise handelt es sich allerdings um eine Metalllegierung mit Formgedächtniseigenschaften. Besonders bevorzugt sind Nickel-Titan-Legierungen, beispielsweise eine Nitinol-Legierung. Die Herstellung von Implantaten aus diesen Materialien und die Umformung durch Tempern sind vielfach beschrieben. Derartige Formgedächtnismetalle können unter äußerem Zwang verformt werden und bei Wegfall dieses Zwangs eine aufgeprägte Form erneut von selbst einnehmen. Dies erlaubt die Verformung des Okkluders in eine Einführlage, um diesen mittels eines Katheters in seine Bestimmungsposition zu bringen und sodann den Okkluder freizusetzen, wobei dieser sich selbst expandiert und dabei seine ihm aufgeprägte Form einnimmt bzw. in seine ihm aufgeprägte Form drängt.

Eine vorteilhafte Weiterbildung der Erfindung sieht vor, dass es sich bei dem biologischen Gewebe um tierisches Gewebe, insbesondere um Gewebe eines Säugetiers, handelt. Ein derartiges Gewebe ist besonders geeignet, zur Anordnung an einem Rahmen eines Okkluders. Insbesondere weist ein derartiges Gewebe eine vergleichsweise hohe Biokompatibilität mit dem Gewebe eines menschlichen Patienten auf.

Eine weitere vorteilhafte Ausgestaltung der Erfindung sieht vor, dass das Gewebe als Pericardium Membran ausgebildet ist. Eine derartige Membran hat sich als ganz besonders vorteilhaft hinsichtlich der Biokompatibilität sowie dem zuverlässigen fluiddichten Verschließen der auricula cordis sinistra herausgestellt. Denkbar wäre allerdings auch jegliches anderes tierisches Gewebe in Membranformat, wie beispielsweise Peritoneumgewebe, Zwerchfellgewebe oder Submukosagewebe des Dünndarms oder Herzbeutelgewebe.

Vorteilhaft ist auch, wenn der Okkluder im expandierten Zustand eine im Wesentlichen kugelförmige Außenkontur aufweist. Eine derartige kugelförmige bzw. ballförmige Außenkontur ermöglicht eine besonders vorteilhafte Positionierung des Okkluders in der auricula cordis sinistra, um diese zu verschließen. Dies insbesondere unabhängig davon, wie genau der Okkluder bzw. der Einführkatheter zum Anordnen des Okkluders relativ zur auricula cordis sinistra im Patienten bei der Freisetzung angeordnet ist und insbesondere unabhängig von der Größe und Kontur der auricula cordis sinistra. Folglich wird dem selbstexpandierbaren Okkluder in diesem Fall insbesondere eine derartige Form aufgeprägt, dass dieser in der expandierten Konfiguration in eine kugelförmige bzw. im Wesentlichen kugelförmige Außenkontur strebt und dabei im Zuge der Freisetzung des Okkluders an den Innenwänden der auricula cordis sinistra zur Anlage kommt. Dabei kann der Okkluder beispielsweise entlang einer Kreislinie an der Innenseite der auricula cordis sinistra zur Anlage kommen.

Besonders bevorzugt ist, wenn das biologische Gewebe im angeordneten Zustand des Okkluders die proximale Hemisphäre der Außenseite des Rahmens wenigstens abschnittsweise, insbesondere wenigstens zur Hälfte, weiter insbesondere wenigstens zu zwei Dritteln, weiter insbesondere vollständig oder im Wesentlichen vollständig, bedeckt. Dadurch kann die auricula cordis sinistra mittels des biologischen Gewebes verschlossen werden, um so insbesondere das Risiko einer Thrombenbildung in der auricula cordis sinistra und der daraus resultierenden Schlaganfallgefahr zu reduzieren. Denkbar ist dabei, dass der Okkluder im angeordneten Zustand entlang einer Kreislinie an der auricula cordis sinistra anliegt. Davon ausgehend in proximaler Richtung ist insbesondere denkbar, dass die Außenseite des Rahmens vollständig oder im Wesentlichen von biologischem Gewebe bedeckt ist, so dass lediglich dieses der Blutzirkulation durch die Aorta ausgesetzt ist. Denkbar ist ferner, dass nach der Anordnung des Okkluders vom Körper des Patienten eine Hautschicht auf dem biologischen Gewebe ausgebildet wird, sodass schlussendlich eine neu gebildete Hautschicht auf dem biologischen Gewebe ausgebildet wird und die auricula cordis sinistra so verschlossen wird.

Besonders bevorzugt ist dabei, wenn das biologische Gewebe am Rahmen, insbesondere mittels PTFE-Fäden, festgenäht ist. Denkbar wäre auch eine Befestigung mittels anderer Befestigungsmethoden. Ein Festnähen an der Rahmenstruktur ist allerdings besonders einfach möglich. Hierzu eignen sich beispielsweise chirurgische Nähte, insbesondere Nähte aus PTFE.

Besonders bevorzugt ist weiter, wenn der Rahmen zum Einführen des Okkluders in einen Patienten in eine Einführlage überführbar ist, in der der Okkluder eine im Wesentlichen rohrförmige Außenkontur aufweist. In diesem Zustand weist der Okkluder folglich insbesondere einen reduzierten Außendurchmesser auf und kann so an die Bestimmungsposition, insbesondere mittels eines Einführkatheters, herangeführt werden. Insbesondere ausgehend von einer dem Okkluder aufgeprägten Form kann dieser folglich in eine komprimierte Konfiguration überführt werden und nach der Freisetzung aus dem Einführkatheter in seine ihm aufgeprägte selbstexpandierte Konfiguration streben und dabei zur Anlage an die Innenseite der auricula cordis sinistra gelangen.

Vorteilhafterweise ist/sind am Rahmen wenigstens ein, insbesondere eine Vielzahl, Röntgenmarker angeordnet. Dies ermöglicht eine besonders positionsgenaue Positionierung des Okkluders an der auricula cordis sinistra und eine darauffolgende Freisetzung des Okkluders. Die Röntgenmarker können insbesondere in gleichmäßigem Abstand entlang der Außenseite des Okkluders angeordnet sein. Insbesondere können diese dabei entlang eines Umfangs des insbesondere kugelförmigen Okkluders angeordnet sein. Dabei kann der Bereich, in dem die Röntgenmarker angeordnet sind, im angeordneten Zustand des Okkluders an der Innenwandung der auricula cordis sinistra zur Anlage kommen. Die Röntgenmarker können folglich insbesondere von einer Schnittebene geschnitten werden, die im angeordneten Zustand des Okkluders die proximale Hemisphäre von der distalen Hemisphäre trennt. Denkbar ist, dass das biologische Gewebe auch die Röntgenmarke abdeckt.

Vorzugsweise umfasst der Rahmen an seinem proximalen Ende einen rohrförmigen Endabschnitt. Im angeordneten Zustand des Okkluders liegt der rohrförmige Endabschnitt folglich im Bereich des proximalen Endes des Rahmens. Durch diesen rohrförmigen Endabschnitt kann insbesondere ein Einführkatheter zur Anordnung des Okkluders an der auricula cordis sinistra in den Rahmen eingeführt werden. Dabei ist denkbar, dass das biologische Gewebe eine Öffnung aufweist, so dass der Einführkatheter durch die Öffnung und sodann durch den rohrförmigen Endabschnitt in den Rahmen einführbar ist. Das biologische Gewebe kann derart elastisch nachgiebig ausgebildet sein, dass nach dem Entfernen des Einführkatheters aus dem Okkluder die Öffnung im Gewebe fluiddicht oder im Wesentlichen fluiddicht verschlossen wird, und somit schlussendlich die auricula cordis sinistra fluiddicht oder im Wesentlichen fluiddicht verschlossen werden kann.

In diesem Zusammenhang ergibt sich eine besonders bevorzugte Weiterbildung der Erfindung daraus, dass der Rahmen an seinem distalen Ende einen topfförmigen Endabschnitt umfasst. Dieser topfförmige Endabschnitt kann demnach einen rohrförmigen Rahmenabschnitt und einen daran anschließenden Bodenabschnitt aufweisen, um so im Bereich des distalen Endes des Okkluders im angeordneten Zustand an der auricula cordis einen topfförmigen distalen Endabschnitt auszubilden. In bzw. an diesem topfförmigen Endabschnitt kann ein Abschnitt eines Einführkatheters anordenbar sein, um den Okkluder so an seine Bestimmungsposition an die auricula cordis sinistra heranzuführen.

Eine besonders bevorzugte Weiterbildung der Erfindung sieht in diesem Zusammenhang ferner vor, dass der Rahmen zwischen dem rohrförmigen Endabschnitt und dem topfförmigen Endabschnitt eine Vielzahl sich verzweigender und wieder zusammenlaufender Stege zur Ausbildung eines netzartigen Rahmenabschnitts umfasst. Zwischen dem proximalen und distalen Endabschnitt des Okkluders kann folglich ein netz- bzw. rahmenartiger Rahmenabschnitt vorgesehen sein. Dieser Rahmenabschnitt kann in der expandierten Konfiguration eine Kugelform oder im Wesentlichen eine Kugelform einnehmen.

Besonders bevorzugt ist dabei, wenn der Rahmen einstückig ausgebildet ist. Der Rahmen kann folglich insbesondere einen distalen und proximalen Endabschnitt sowie einen dazwischen liegenden netzartigen Rahmenabschnitt umfassen. Dabei kann der einstückige Rahmen hergestellt sein durch Laserschneiden, mittels eines Hydrojet-Verfahrens oder mittels Funkenerodieren (englisch: electrical discharge machining EDM). Folglich kann insbesondere die Vielzahl sich verzweigender und wieder zusammenlaufender Stege aus einem rohrförmigen Abschnitt "herausgeschnitten" werden. Denkbar wäre allerdings auch, dass die netzartige Rahmenstruktur aus einer geflochtenen Struktur hergestellt wird.

Vorteilhafterweise weist die proximale Hemisphäre des Okkluders erste Verankerungsmittel auf. Zusätzlich oder alternativ weist die distale Hemisphäre zweite Verankerungsmittel auf.

Im angeordneten Zustand des Okkluders kann die proximale Hemisphäre folglich erste Verankerungsmittel aufweisen. Diese können insbesondere als Haken (englischer Fachausdruck: hooks) ausgebildet sein und eine gebogene Struktur aufweisen mit einem Endabschnitt, der in proximale Richtung weist.

Zusätzlich oder alternativ kann die distale Hemisphäre zweite Verankerungsmittel aufweisen. Diese können insbesondere als Haken (englischer Fachausdruck: barbs) ausgebildet sein und dabei insbesondere einen stabförmigen Abschnitt umfassen, der in proximale Richtung weist.

Vorzugsweise ist das biologische Gewebe stabilisiert durch Behandlung mittels einer Vernetzungsmethode vor der Anordnung am Rahmen. Denkbar ist folglich, dass das biologische Gewebe zunächst mittels eines cross-link-Verfahrens stabilisiert wird, beispielsweise unter Verwendung von Glutaraldehyd und/oder Alkohol. Denkbar ist, dass das biologische Gewebe, insbesondere nachdem es am Okkluder befestigt wurde, sodann in flüssigem Medium aufbewahrt wird, bis der Okkluder verwendet werden soll und erst unmittelbar vor der Anordnung im Patienten samt Okkluder am Katheter angeordnet wird. Denkbar wäre andererseits allerdings insbesondere auch, dass das biologische Gewebe getrocknet wird und so haltbar gemacht wird. Dabei ist denkbar, dass dieses zunächst mittels Glycerol und einem ein- bis dreiwertigen Alkohol behandelt wird. Danach kann das Gewebe getrocknet und insbesondere vakuumgetrocknet werden. Denkbar wäre auch jegliches anderes Gewebetrocknungsverfahren zur Haltbarmachung des biologischen Gewebes. Ein derart getrocknetes Gewebe kann folglich am Rahmen des Okkluders angeordnet werden. Sodann kann der Okkluder in diesem Zustand in oder an dem Einführkatheter angeordnet werden. Daraufhin kann das System aus Einführkatheter und Okkluder, umfassend das biologische Gewebe, mittels insbesondere gasförmigem Ethylenoxid (EtO) sterilisiert werden und in dieser Konfiguration während der Haltbarkeitsdauer haltbar gelagert werden.

Die eingangs gestellte Aufgabe wird auch gelöst durch ein System zur Einführung eines Okkluders in einen Patienten und zum Anordnen des Okkluders an der auricula cordis sinistra des Patienten, das System umfassend einen Einführkathether und einen daran angeordneten erfindungsgemäßen Okkluder, sowie ein Verfahren zur Bereitstellung des Systems. Mittels des Systems kann der Okkluder folglich der auricula cordis sinistra zugeführt und sodann dort freigesetzt werden. Dabei ist insbesondere denkbar, dass der Okkluder einen rohrförmigen proximalen Endabschnitt aufweist, durch den der Einführkatheter in den Okkluder eingeführt ist. Der Einführkatheter kann insbesondere einen Außenschlauch und einen Innenschlauch aufweisen. Der Außenschlauch kann dabei in distaler Richtung vor dem Innenschlauch enden. Der Außenschlauch kann mit dem proximalen Endabschnitt des Okkluders bewegungsgekoppelt sein, während der Innenschlauch mit dem distalen Endabschnitt des Okkluders bewegungsgekoppelt sein kann. Der distale Endbereich des Innenschlauchs kann in den distalen topfförmigen Endabschnitt des Okkluders eingeführt sein. Dabei ist denkbar, dass Innenschlauch und Außenschlauch relativ zueinander verlagerbar sind, um so den Okkluder zunächst in einen gestreckten Zustand, also eine Einführlage, zu überführen, in dem dieser insbesondere eine rohrförmige Konfiguration aufweisen kann und den Okkluder sodann an Bestimmungsort an der auricula cordis sinistra zu verbringen. Daraufhin kann der Okkluder freigesetzt werden und in seine, insbesondere kugelförmige, selbstexpandierte Konfiguration drängen und dabei zur Anlage an der auricula cordis sinistra gelangen.

Es ist zudem vorteilhaft, wenn der Okkluder nach dem Aufbringen des biologischen Gewebes auf den Rahmen zur Lagerung und/oder zum Transport zur Bedienperson bereits innerhalb des Einführkatheters angeordnet ist (pre-loaded system). Durch die Sterilisierung des Systems aus dem Einführkatheter und dem Okkluder mittels insbesondere Ethylenoxid (ETO) kann das System haltbar gelagert und transportiert werden. Dabei ergeben sich ferner Vorteile unmittelbar vor dem Einbringen des Systems in den Patienten, da der Okkluder nicht separat zum Einführkatheter als Einzelteil vorliegt und damit die Bedienperson nicht den Okkluder an den Einführkatheter anordnen muss. Somit kann durch diese Ausführungsform ein Arbeitsschritt eingespart werden und folglich besteht ein geringeres Risiko hinsichtlich Verunreinigungen oder Bedienfehler.

Das Verfahren zum Bereitstellen eines erfindungsgemäßen Systems sieht vor, dass zunächst biologisches Gewebe durch eine Vernetzungsmethode stabilisiert wird, und wobei dann das biologische Gewebe zur Sterilisation und Haltbarmachung mit einem flüssigen Medium benässt und/oder getrocknet wird. Das biologische Gewebe kann getrocknet werden, insbesondere mittels einer Vakuumtrocknung, dann am Okkluder angeordnet und in den Einführschlauch eingebracht werden (dry-state). Das gesamte System mit im Einführschlauch vorhandenem Okkluder kann dann der Bedienperson, insbesondere dem Arzt, Verfügung gestellt werden (pre-loaded system). Allerdings ist auch denkbar, dass der Okkluder mit dem biologischen Gewebe in einem sterilisierenden Flüssigkeitsbad der Bedienperson getrennt vom Einführkatheter zur Verfügung gestellt wird (wetstate), und dass unmittelbar vor der Benutzung des Systems der Okkluder am Einführkatheter befestigt wird (user-loaded system).

Weitere Einzelheiten und vorteilhafte Ausgestaltungen der Erfindung sind der folgenden Beschreibung zu entnehmen, anhand derer die in den Figuren dargestellte Ausführungsform der Erfindung näher beschrieben und erläutert ist.

Es zeigen:
- Figur 1: schematische Draufsicht auf einen an der auricula cordis sinistra angeordneten Okkluder gemäß einer Ausführungsform;
- Figur 2: schematische perspektivische Darstellung des Okkluders gemäß Figur 1;

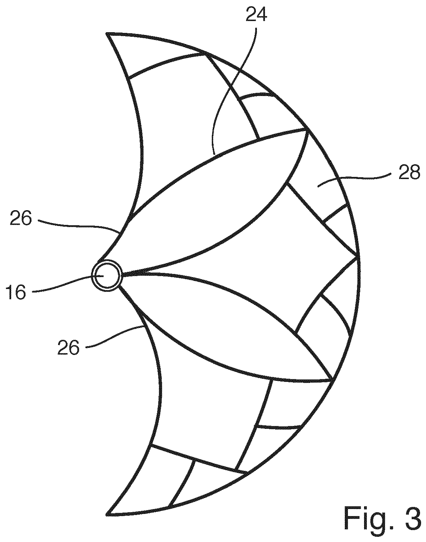

- Figur 3: Draufsicht auf eine Hälfte des Okkluders gemäß Figur 2;
- Figur 4: aufgeschnittene schematische Darstellung eines Rahmenabschnitts des Okkluders gemäß Figur 2;
- Figur 5: perspektivische schematische Darstellung einer Einführeinheit gemäß einer Ausführungsform;
- Figur 6: schematische Querschnittsdarstellung der Einführeinheit gemäß Figur 1 mit einem in einem Einführschlauch angeordneten Okkluder in Einführlage;
- Figur 7: schematische Querschnittsdarstellung entsprechend Figur 6 ohne Einführschlauch;
- Figur 8: schematischer Querschnitt der Einführeinheit gemäß Figur 1 mit daran angeordnetem Okkluder in einer Konfiguration vor dem Entfernen der Einführeinheit vom Okkluder.
- Figur 9: schematischer Querschnitt eines Bereichs um das proximale Ende des Okkluders herum mit daran angeordneter Einführeinheit;
- Figur 10: Darstellung basierend auf Figur 9, wobei der Innenschlauch der Einführeinheit vom Okkluder entfernt ist;
- Figur 11: Darstellung eines proximalen Bereichs der Einführeinheit mit von der Einführeinheit gelöstem Luer-Anschluss; und
- Figur 12: Verfahrensschritte zur Bereitstellung eines erfindungsgemäßen Systems.

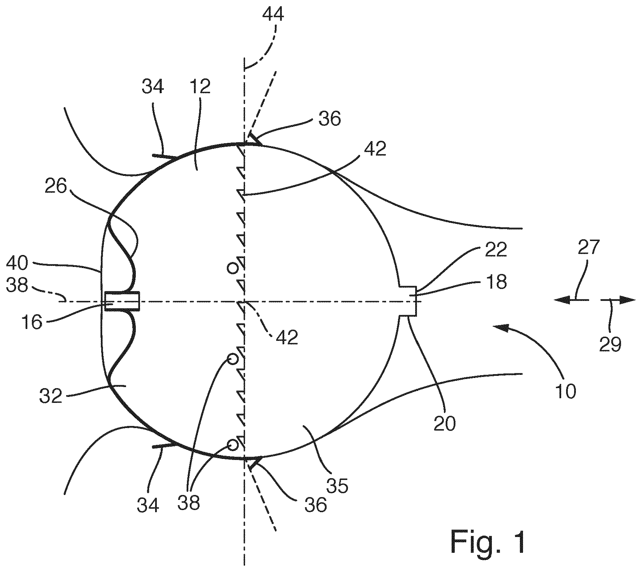

Figur 1 zeigt mit dem Bezugszeichen 10 zunächst schematisch die auricula cordis sinistra eines Patienten. Zur Reduzierung des Schlaganfallrisikos ist dabei ein Okkluder 12 in die auricula cordis sinistra 10 eingesetzt, um den Zugang zur auricula cordis sinistra 10 zu verschließen.

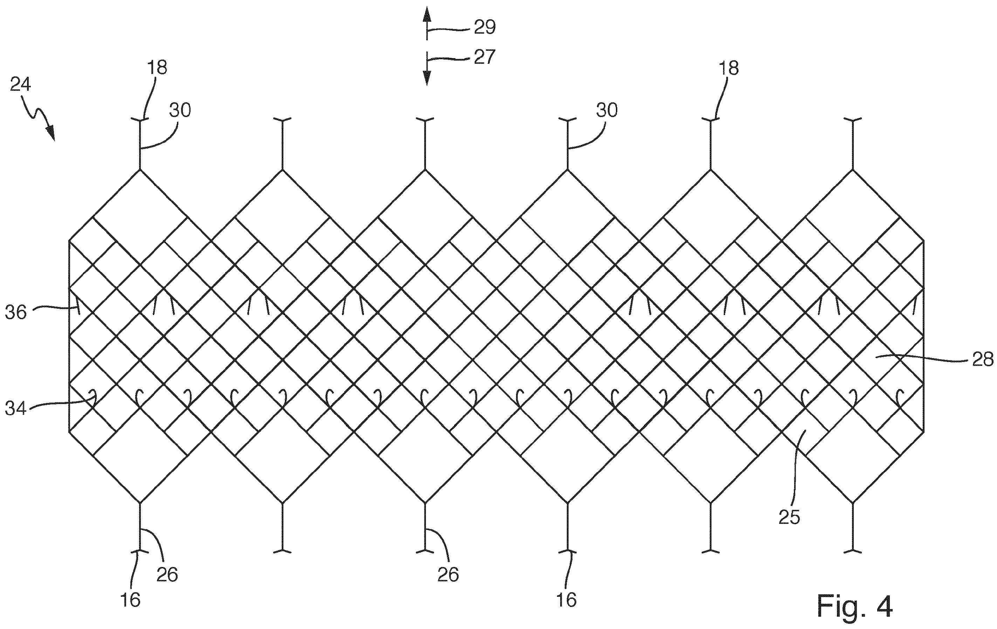

Der Okkluder 12 umfasst zunächst einen einstückig ausgebildeten Rahmen 14. Dieser umfasst einen proximalen rohrförmigen Abschnitt 16 und einen topfförmigen distalen Endabschnitt 18. Der topfförmige distale Endabschnitt 18 umfasst einen kreiszylindrischen Mantelabschnitt 20 und einen Bodenabschnitt 22, um so eine topfförmige Struktur auszubilden. Zwischen den beiden Endabschnitten 16, 18 weist der Okkluder einen netzartigen Rahmenabschnitt 24 auf. Dieser netzartige Rahmenabschnitt 24 ist in Figur 4 in aufgeschnittener Form besonders gut ersichtlich. Ausgehend vom proximalen rohrförmigen Endabschnitt 16 weist der netzartige Rahmenabschnitt 24 zunächst eine Anzahl Stege 26 auf. Diese gehen in ein verzweigendes Netzwerk an Stegen 25 über, um so die Netzstruktur auszubilden. Dabei werden rautenförmige Strukturen 28 ausgebildet. In distaler Richtung 29 laufen die Stege 25 wieder zusammen in einzelne Stege 30, die in den distalen Endabschnitt 18 münden.

Im angeordneten Zustand (vgl. Figur 1) weist der Okkluder 12 eine proximale Hemisphäre 32 und eine distale Hemisphäre 35 auf. Im Bereich der proximalen Hemisphäre 32 sind eine Anzahl erste Verankerungsmittel 34 vorgesehen. Diese erstrecken sich entlang einer Kreislinie entlang des Umfangs und sind hakenförmig ausgebildet mit Endabschnitten, die in proximale Richtung 27 weisen. Im Bereich der distalen Hemisphäre 35 sind zweite Verankerungsmittel 36 ausgebildet. Diese erstrecken sich ebenfalls entlang einer Kreislinie entlang des Umfangs, weisen eine stabartige Form auf und ragen schräg von der Umfangsfläche in proximale Richtung 27 ab. Die Verankerungsmittel 34, 36 sind ebenfalls einstückig mit dem Rahmen 14 ausgebildet.

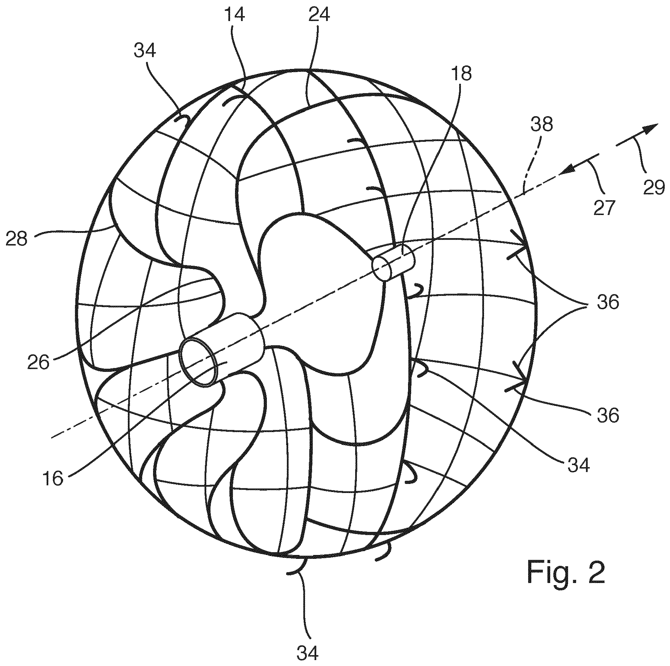

Der Rahmen 14 des Okkluders 12 besteht aus einem selbstexpandierbaren Material, beispielsweise aus einer Formgedächtnislegierung, insbesondere aus einer Nitinol-Legierung. Die dem Okkluder 12 aufgeprägte expandierte Form ist kugelförmig (vgl. Figur 2). Dabei weist der Okkluder 12 eine in proximale bzw. distale Richtung verlaufende Längsachse 38 durch seinen Mittelpunkt auf. Diese Längsachse 38 erstreckt sich auch durch die Mittellängsachse des rohrförmigen proximalen Endabschnitts 16 sowie durch die Mittellängsachse des distalen Endabschnitts 18 (vgl. Figur 2). Die proximale Hemisphäre 32 ist vollständig von einem biologischen Gewebe 40 bedeckt. Dieses biologische Gewebe 40 ist insbesondere als biologische Membran ausgebildet. Insbesondere kann es sich um die Pericardium Membran handeln. Das Gewebe 40 weist Öffnungen auf, sodass die ersten Verankerungsmittel 34 durch die Öffnungen hindurchragen. Ferner weist das Gewebe 40 eine Einführöffnung auf, um einen Einführkatheter durch den proximalen Rohrabschnitt 16 in den Okkluder 12 einzuführen. Wenn der Einführkatheter nach der Freisetzung des Okkluders 12 aus dem Okkluder 12 entfernt worden ist, kann das elastisch nachgiebige Gewebe 40 sich so zusammenziehen, dass die Einführöffnung im Wesentlichen fluiddicht verschlossen wird, so dass insgesamt das Gewebe 40 die proximale Hemisphäre 32 im Wesentlichen fluiddicht abschließt und den Rahmen 14 dabei im Wesentlichen abdeckt. Wie schematisch durch chirurgische Fäden 42 angedeutet, ist das Gewebe mittels PTFE-Fäden am Rahmen 14 angenäht.

In der Nähe der Trennebene 44 der proximalen Hemisphäre 32 und der distalen Hemisphäre 35 sind im Bereich der proximalen Hemisphäre 32 über den Umfang eine Anzahl Röntgenmarker 38 platziert. Diese ermöglichen eine exakte Positionierung des Okkluders 12 in der auricula cordis sinistra. Ein Chirurg kann folglich eine besonders positionsgenaue Platzierung des Okkluders 12 durchführen.

Insgesamt kann durch das Vorsehen des biologischen Gewebes 40 ein Okkluder 12 mit vergleichsweise hoher Biokompatibilität bereitgestellt werden. Dabei kann das biologische Gewebe 40 nach der Anordnung des Okkluders 12 an der auricula cordis sinistra von natürlichem Gewebe des Patienten überwachsen werden. Aufgrund des verwendeten biologischen Gewebes 40 besteht insgesamt eine hohe Biokompatibilität und damit eine erhöhte Erfolgswahrscheinlichkeit, dass ein chirurgischer Eingriff zum Verschließen der auricula cordis sinistra erfolgreich ist.

Im Folgenden wird ein System zum Einführen des Okkluders 12 in einen Patienten und zum Freisetzen des Okkluders 12 in der auricula cordis sinistra 10 des Patienten gemäß einer Ausführungsform dargelegt:

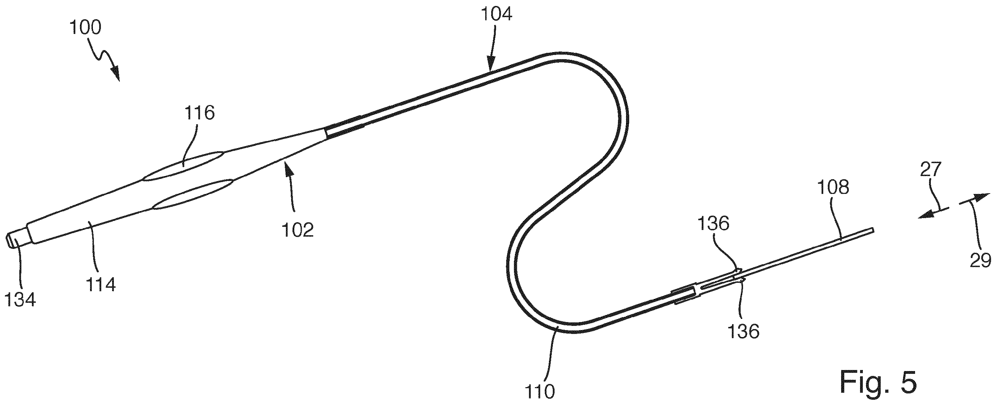

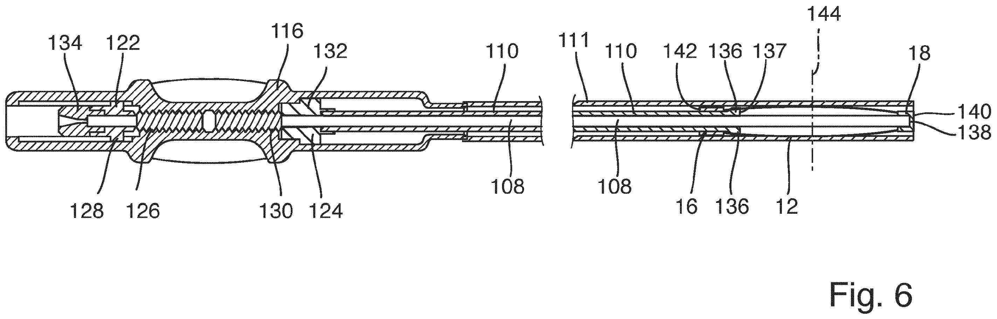

Figur 5 zeigt insgesamt eine Einführeinheit 100. Diese umfasst zum einen eine Antriebseinheit 102 und zum anderen einen Einführkatheter 104. Der Einführkatheter 104 umfasst einen Innenschlauch 108 und einen Außenschlauch 110. Dabei erstreckt sich der Innenschlauch 108 durch den Außenschlauch 110. Ferner endet der Außenschlauch 110 in distaler Richtung 29 vor dem Innenschlauch 108. Der Einführkatheter 104 kann zudem, wie in Figur 6 gezeigt ist, einen den Außenschlauch 110 umgebenden Einführschlauch 111 aufweisen. Die Antriebseinheit 102 umfasst ein Gehäuse 114, das von einer Bedienperson, insbesondere einem Chirurg, in der Hand gehalten werden kann und insgesamt eine längliche Form aufweist.

Ferner umfasst die Antriebseinheit 102 ein Betätigungselement 116, das drehbar im Gehäuse 114 angeordnet ist. Dabei ist das Betätigungselement 116 hohl ausgebildet mit einem ersten Antriebsgewinde 118 und einem zweiten Antriebsgewinde 120, wobei das erste Antriebsgewinde 118 proximal zum zweiten Antriebsgewinde 120 ist (vgl. Figuren 6, 7 und 8). Das Betätigungselement 116 ist insbesondere einstückig ausgebildet.

Im Gehäuse 114 sind ferner ein erstes Übertragungselement 122 und ein zweites Übertragungselement 124 angeordnet. Beide Übertragungselemente 122, 124 weisen insgesamt eine schraubenartige Form auf. Dabei weist das erste Übertragungselement 122 einen Gewindeabschnitt 126 und einen Kopfabschnitt 128 auf. Dementsprechend weist das zweite Übertragungselement 124 einen Gewindeabschnitt 130 und einen Kopfabschnitt 132 auf. Beide Übertragungselemente 122, 124 können insbesondere jeweils einstückig ausgebildet sein. Das erste Übertragungselement 122 ist dabei mit dem Innenschlauch 108 bewegungsgekoppelt, während das zweite Übertragungselement 124 mit dem Außenschlauch 110 bewegungsgekoppelt ist. Der Kopfabschnitt 128 des ersten Übertragungselements 122 wirkt dabei mit einem Luer-Anschluss 134 zusammen, der am Innenschlauch 108 befestigt ist. Dabei ist der Luer-Anschluss 134 am Kopfabschnitt 128 des ersten Übertragungselements 122 lösbar angeordnet.

Der Kopfabschnitt 132 des zweiten Übertragungselements 124 wirkt mit dem Außenschlauch 110 zur Bewegungskopplung zusammen. Dabei ist der Außenschlauch 110 lösbar am Kopfabschnitt 132 angeordnet.

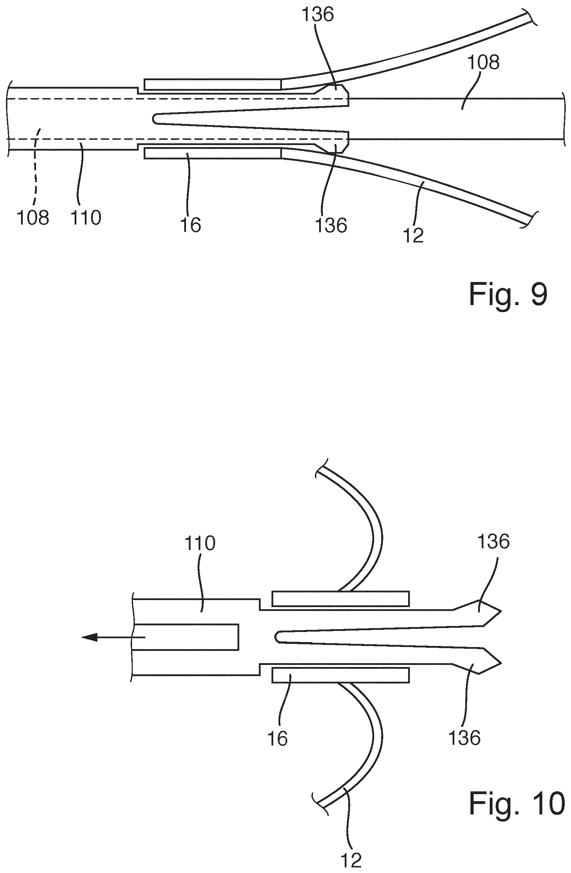

Am distalen Ende des Außenschlauchs 110 weist dieser zwei in Figur 10 besonders deutlich zu erkennende rastfingerartige Endabschnitte 136 auf. Diese sind einstückig mit dem Außenschlauch 110 ausgebildet und elastisch nachgiebig verformbar. Insgesamt ist die Funktionsweise der Einführeinheit 100 sodann wie folgt:
Um den Okkluder 12 in die auricula cordis sinistra 10 einzuführen, wird zunächst der Einführkatheter 104 an dem Okkluder 12 angeordnet, um so ein System aus Einführeinheit 100 und Okkluder 12 auszubilden, um den Okkluder 12 an die auricula cordis sinistra 10 heranzuführen und den Okkluder 12 sodann freizusetzen.

Der Okkluder 12 befindet sich dabei zunächst in seiner selbstexpandierten Form und weist somit eine kugelförmige Außenkontur auf (vgl. Figur 2). Dabei wird der Außenschlauch 110 durch eine nicht gezeigte Einführöffnung im biologischen Gewebe 40 des Okkluders 12 sowie durch den rohrförmigen proximalen Rohrabschnitt 16 in den Okkluder 12 eingeführt. Sodann wird der Innenschlauch 108 durch den Okkluder 12 und den Außenschlauch 110 hindurchgeführt und am distalen topfförmigen Endabschnitt 18 des Okkluders 12 angeordnet. Dabei werden die rastfingerartigen Abschnitte 136 vom Innenschlauch 108 gegen eine elastische Verformung nach radial innen gesichert, so dass die Rastfinger 136 formschlüssig am proximalen rohrförmigen Abschnitt 16 zur Anlage kommen.

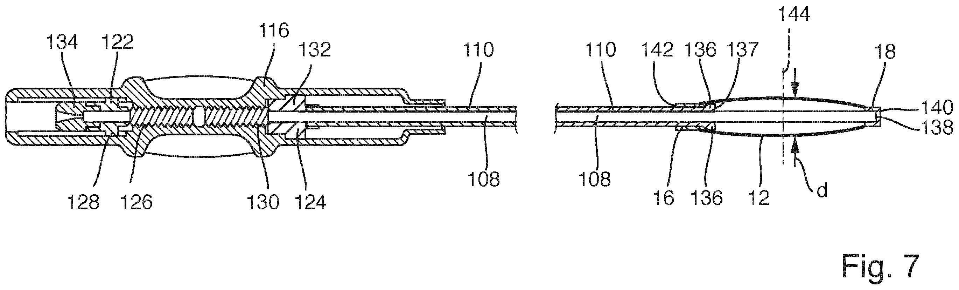

Sodann kann von einer Bedienperson, insbesondere von einem Chirurgen, das Gehäuse 114 in die Hand genommen werden und das Betätigungselement 116 rotiert werden. Wie in Figur 7 gezeigt, wird dabei das Betätigungselement 116 zunächst so rotiert, dass die kopfartigen Abschnitte 128, 132 der beiden Übertragungselemente 122, 124 aufeinander zu bewegt werden. Dadurch werden das distale Ende 137 des Außenschlauchs 110 sowie das distale Ende 138 des Innenschlauchs 108 voneinander weg bewegt. Dadurch wird das distale Ende 140 des Okkluders 12 in distaler Richtung 29 verlagert, während das proximale Ende 142 des Okkluders in proximaler Richtung 27 verlagert wird. Insgesamt wird dabei der Okkluder 12 in eine Einführlage überführt, so dass der Okkluder 12 insgesamt eine komprimierte Form einnimmt, in der dieser gegenüber der selbstexpandierten Form eine längliche Außenkontur mit einem verringerten Durchmesser d aufweist (vgl. Figur 7). Der Okkluder wird damit von der Bedienperson selbst in seine Einführlage eingebracht (User-loaded system).

Alternativ ist denkbar, dass der Okkluder 12 am Einführkatheter 104 als vorkonfiguriertes System zur Verfügung gestellt wird; der Okkluder 12 kann dabei vom Einführschlauch 111 geschützt umgeben sein und wird in seiner Einführlage "geladen" zur Verfügung gestellt (pre-loaded system). Der Okkluder 12 kann im Einführschlauch 111 das biologische Gewebe insbesondere haltbar gemacht aufweisen. Das biologische Gewebe kann mit eine flüssige Medium, vorzugsweise in einem Alkohol wie beispielweise Glycerol konditioniert sein, und danach vakuumgetrocket sein. Ferner kann das biologische Gewebe mittels Ethylenoxid (EtO) sterilisiert sein und in dieser Konfiguration im Einführkatheter 104, bzw. dessen Einführschlauch 111 haltbar in der komprimierten Einführlage gelagert werden.

In der komprimierten Einführlage kann - wie oben beschrieben - der Okkluder 12 zusammen mit dem Einführkatheter 104 in ein Blutgefäß eingeführt werden und dann weiter bis zur auricula cordis sinistra an seine Bestimmungsposition herangeführt werden. Die Position des Okkluders 12 kann mittels der Röntgenmarker 38 festgestellt werden. Der Okkluder 12 soll dabei insbesondere im mittleren Bereich, also im Bereich der Röntgenmarker 38, an der auricula cordis sinistra zur Anlage kommen.

Für den Fall, dass der Einführkatheter 104 den Einführschlauch 111 umfasst, wird dieser gegenüber dem Außenschlauch 110 zurückgezogen, wenn der Okkluder 12 seine Position an der auricula cordis sinistra 10 erreicht hat, wodurch der Okkluder freigesetzt werden kann.

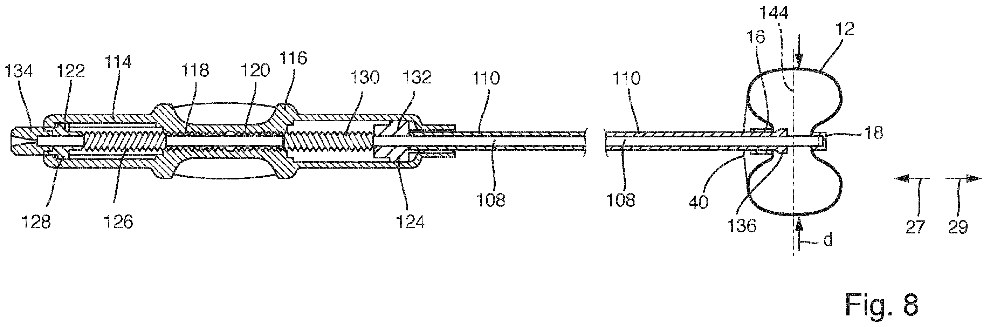

Um den Okkluder 12 vollends freizusetzen, wird, wie in Figur 8 gezeigt, das Betätigungselement 116 derart rotiert, sodass die kopfartigen Abschnitte 128, 132 voneinander weg bewegt werden. Dadurch wird der Innenschlauch 108 in proximaler Richtung 27 bewegt, während der Außenschlauch 110 in distaler Richtung 29 bewegt wird. Im Zuge dieser Relativbewegung werden das proximale Ende 142 des Okkluders 12 und das distale Ende 140 des Okkluders 12 aufeinander zu bewegt. Dadurch wird der Durchmesser d des Okkluders 12 größer. Der Okkluder 12 drängt dabei in seine selbstexpandierte Form, sodass das proximale Ende 142 und das distale Ende 140 des Okkluders aufeinander zu drängen.

Die Position des mittleren Teils des Okkluders 12 bleibt dabei insbesondere unverändert, was in den Figuren 7 und 8 anhand der Mittellinie 144 dargestellt ist. Die Mittellinie 144 verläuft dabei durch die Mitte des Okkluders 12 zwischen dem proximalen Ende 142 und dem distalen Ende 140, wenn dieser, wie in Figur 7 gezeigt, an der Bestimmungsposition angelangt. Die Mitte des Okkluders 12 bleibt also lagegetreu und damit in seiner Lage unverändert, wenn der Okkluder, wie in Figur 8 dargestellt, freigesetzt wird.

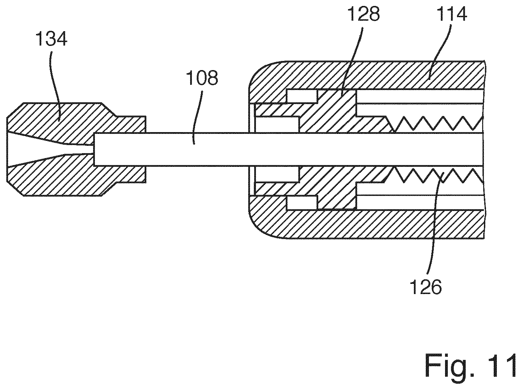

In der in Figur 7 gezeigten Lage ist der Innenschlauch 108 am topfförmigen distalen Endabschnitt 18 des Okkluders 12 kraftschlüssig in proximaler Richtung 27 gehalten. Folglich wird bei einer proximalen Bewegung des Innenschlauchs 108 ebenfalls der distale Endabschnitt 18 des Okkluders 12 in proximaler Richtung 27 bewegt. Wenn der Okkluder 12 dabei in eine Lage, wie in Figur 8 gezeigt, überführt ist, ist dabei die Kraft des Kraftschlusses reduziert, so dass, wie in Figur 11 gezeigt, der Luer-Anschluss 134 vom kopfartigen Abschnitt 128 mit vergleichsweise geringem Kraftaufwand lösbar ist, so dass der Luer-Anschluss 134 und damit der Innenschlauch 108 vom Okkluder 12 durch den Außenschlauch 110 hindurch abziehbar ist.

Daraufhin kann, wie in Figur 11 gezeigt, der Außenschlauch 110 vom Okkluder 12 abgezogen werden, da sich die rastfingerartigen Abschnitte 136 nunmehr elastisch nach radial innen verlagern können. Sodann kann der Okkluder 12 seine Freigabelage einnehmen, in der dieser seine selbstexpandierte Form, wie in Figur 1 gezeigt, einnehmen kann oder jedenfalls in seine selbstexpandierte Endform drängt und damit die auricula cordis sinistra dicht verschließen kann. Wenn der Einführkatheter nach der Freisetzung des Okkluders 12 aus dem Okkluder 12 entfernt worden ist, kann das elastisch nachgiebige Gewebe 40 sich so zusammenziehen, dass die nicht gezeigte Einführöffnung im Gewebe 40 im Wesentlichen fluiddicht verschlossen wird, so dass insgesamt das Gewebe 40 die proximale Hemisphäre 32 im Wesentlichen oder vollständig fluiddicht abschließt und den Rahmen 14 dabei im Wesentlichen oder vollständig abdeckt. Sodann kann Haut des Patienten über das biologische Gewebe 40 wachsen, sodass die auricula cordis sinistra dauerhaft und stabil verschlossen werden kann. Aufgrund der vorgeschlagenen Konfiguration kann der Einführkatheter 104 besonders einfach vom Okkluder 12 abgezogen werden. Dies insbesondere ohne, oder nahezu ohne, dass ein Drehmoment auf den Okkluder 12 ausgeübt wird. Somit ist die Gefahr reduziert, dass beim Abziehen des Einführkatheters 104 vom Okkluder 12 dieser von seiner Bestimmungsposition in unerwünschter Weise verlagert wird.

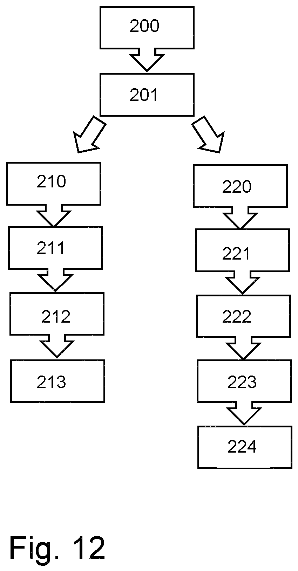

Das Verfahren zum Bereitstellen eines erfindungsgemäßen Systems ist in Figur 12 schematisch wiedergegeben und sieht folgende Schritte vor:
Im Verfahrensschritte 200 wird zunächst biologisches Material, insbesondere Pericardium, bereitgestellt und in Verfahrensschritte 201 durch eine Vernetzungsmethode (Crosslinking) insbesondere mittels Glutaraldehyd und/oder Alkohol stabilisiert wird.

In einem nächsten Verfahrensschritt 210 kann dann das vernetzte biologische Material an den Okkluder, bzw. an dessen Rahmen befestigt werden und insbesondere angenäht werden. In dem sich daran anschließenden Verfahrensschritte 211 wird der Okkluder mit dem biologischen Material in einem flüssigen Medium präserviert und damit haltbar gemacht. Das flüssige Medium kann dabei Glutaraldehyd und/oder ein Alkohol sein. Im Verfahrensschritte 212 wird der Einführkatheter, unabhängig vom Okkluder, sterilisiert, insbesondere mit gasförmigem Ethylenoxid (EtO). Der Okkluder und der Einführkatheter können dann getrennt voneinander verpackt und für den Arzt zur Verfügung gestellt werden. Im Verfahrensschritte 213 wird dann, in der Regel im Operationsraum, der benässte Okkluder am Einführkatheter montiert bzw. in diesem angeordnet werden (User-loaded System).

An den Verfahrensschritte 201 kann können sich anstelle der Verfahrensschritte 210 bis 213 die Verfahrensschritte 220 bis 224 anschließen. Im Verfahrensschritte 220 wird das biologische Material insbesondere mittels Glycerol und/oder einem ein- bis dreiwertigen Alkohol getrocknet, und zwar insbesondere vakuumgetrocknet oder Luft getrocknet. Im Verfahrensschritte 221 wird das biologische Material an den Okkluder, bzw. dessen Rahmen befestigt, insbesondere angenäht. Im Verfahrensschritt 222 wird der Okkluder samt dem biologischen Material in den Einführkatheter eingebracht. Im Verfahrensschritt 222 wird der Okkluder mit dem biologischen Material innerhalb des Einführkatheters sterilisiert, insbesondere mit gasförmigem Ethylenoxid (EtO). Das so vormontierte System kann dann entsprechend verpackt und gelagert werden und im Verfahrensschritt 224 dem Arzt im Operationsraum als Pre-loaded System zur Verfügung gestellt werden. Das System ist dann "ready to use".

## Patentansprüche

1. Okkluder (12) zum Verschließen der auricula cordis sinistra (10) eines Patienten, umfassend einen selbstexpandierbaren Rahmen (14), **dadurch gekennzeichnet, dass** der Okkluder (12) im expandierten Zustand eine im Wesentlichen kugelförmige Außenkontur aufweist, wobei am Rahmen (14) ein biologisches Gewebe (40) angeordnet ist, das die proximale Hemisphäre (32) der Außenseite des Rahmens (14) wenigstens abschnittsweise bedeckt, dass das Gewebe (40) dazu ausgebildet ist, im angeordneten Zustand des Okkluders (12) die auricula cordis sinistra (10) fluiddicht zu verschließen, und dass das biologische Gewebe (40) durch Behandlung mittels einer Vernetzungsmethode stabilisiert und zur Haltbarmachung, entweder mit einem flüssigen Medium benässt, oder getrocknet ist.

2. Okkluder (12) nach Anspruch 1, **dadurch gekennzeichnet, dass** das biologische Gewebe (40) als Pericardium Membran ausgebildet ist.

3. Okkluder (12) nach einem der vorhergehenden Ansprüche, **dadurch gekennzeichnet, dass** die Vernetzungsmethode das cross-link-Verfahren unter Verwendung von insbesondere Glutaraldehyd und/oder Alkohol umfasst.

4. Okkluder (12) nach einem der Ansprüche 1 bis 3,
**dadurch gekennzeichnet, dass** das flüssige Medium, mit dem das biologische Gewebe (40) benässt ist, Glutaraldehyd und/oder Alkohol ist.

5. Okkluder (12) nach einem der Ansprüche 1 bis 3,
**dadurch gekennzeichnet, dass** das biologische Gewebe (40) mit Glycerol und/oder mit einem ein- bis dreiwertigen Alkohol zunächst benässt und dann getrocknet ist.

6. Okkluder (12) nach Anspruch 5, **dadurch gekennzeichnet, dass** das biologische Gewebe (40) vakuumgetrocknet oder luftgetrocknet ist.

7. Okkluder (12) nach Anspruch 6, **dadurch gekennzeichnet, dass** das getrocknete biologische Gewebe (40) mit Ethylenoxid sterilisiert ist.

8. Okkluder (12) nach einem der vorhergehenden Ansprüche, **dadurch gekennzeichnet, dass** der Okkluder (12) ein proximales Ende (142) und ein distales Ende (140) umfasst, und dass in einer Einführlage der Okkluder (12) gegenüber der selbstexpandierten Form eine längliche Außenkontur aufweist, in der das proximale Ende (142) in proximaler Richtung und das distale Ende (140) in distaler Richtung verlagert ist und der Okkluder (12) gestreckt ist.

9. Okkluder (12) nach einem der vorhergehenden Ansprüche, wobei der Rahmen (14) an seinem proximalen Ende einen rohrförmigen Endabschnitt (16) umfasst und/oder wobei der Rahmen (12) an seinem distalen Ende einen topfförmigen Endabschnitt (18) umfasst.

10. Okkluder (12) nach einem der vorhergehenden Ansprüche, wobei die proximale Hemisphäre (32) erste Verankerungsmittel (34) aufweist, und/oder wobei eine distale Hemisphäre (35) des Okkluders (12) zweite Verankerungsmittel (36) aufweist.

11. System zur Einführung eines Okkluders (12) in einen Patienten und zum Anordnen des Okkluders (12) an der auricula cordis sinistra (10) des Patienten, das System umfassend einen Einführkatheter (104) und einen Okkluder (12) mit biologischem Gewebe (40) nach einem der vorhergehenden Ansprüche.

12. Verfahren zum Bereitstellen eines Systems nach Anspruch 11, wobei zunächst biologisches Gewebe (40) durch eine Vernetzungsmethode stabilisiert wird und zur Haltbarmachung, entweder mit einem flüssigen Medium benässt, oder getrocknet wird.

13. Verfahren nach Anspruch 12, wobei das flüssige Medium Glutaraldehyd und/oder Alkohol umfasst und der Okkluder (12) mit dem biologischen Gewebe (40) unmittelbar vor dessen Einführung in den Patienten am Einführkatheter (104) angeordnet wird.

14. Verfahren nach Anspruch 12, wobei das biologische Gewebe (40) vakuumgetrocknet oder luftgetrocknet wird und wobei der Okkluder (12) mit dem biologischen Gewebe (40) in den Einführkatheter (104) eingebracht und haltbargemacht zur Verfügung gestellt wird.

## Claims

1. Occluder (12) for closing the left atrial appendage (10) of a patient, comprising a self-expandable frame (14), **characterized in that**, in the expanded state, the occluder (12) has a substantially spherical outer contour, wherein a biological tissue (40) is arranged on the frame (14) that at least partially covers the outside of the proximal hemisphere (32) of the frame (14), **in that** the biological tissue (40) is configured to close the left atrial appendage (10) in the arranged state of the occluder (12) in a fluid-tight manner, and **in that** the biological tissue (40) is stabilized by treatment by means of a reticulation method and, in order to make it durable, is either wetted with a liquid medium or dried.

2. Occluder (12) according to claim 1, **characterized in that** the biological tissue (40) is configured as pericardium membrane.

3. Occluder (12) according to any of the preceding claims, **characterized in that** the reticulation method comprises the crosslinking process using in particular glutaraldehyde and/or alcohol.

4. Occluder (12) according to any of claims 1 to 3, **characterized in that** the liquid medium with which the biological tissue (40) is wetted is glutaraldehyde and/or alcohol.

5. Occluder (12) according to any of claims 1 to 3, **characterized in that** the biological tissue (40) is initially wetted with glycerol and/or with a mono- to trihydric alcohol and is then dried.

6. Occluder (12) according to claim 5, **characterized in that** the biological tissue (40) is vacuum-dried or air-dried.

7. Occluder (12) according to claim 6, **characterized in that** the dried biological tissue (40) is sterilized with ethylene oxide.

8. Occluder (12) according to any of the preceding claims, **characterized in that** the occluder (12) comprises a proximal end (142) and a distal end (140), and **in that**, in an insertion position, the occluder (12) has, by comparison with the self-expanded form, an elongate outer contour in which the proximal end (142) is displaced in the proximal direction and the distal end (140) is displaced in the distal direction and the occluder (12) is stretched.

9. Occluder (12) according to any of the preceding claims, wherein the frame (12) comprises a tubular end portion (16) at its proximal end and/or wherein the frame (12) comprises a pot-shaped end portion (18) at its distal end.

10. Occluder (12) according to any of the preceding claims, wherein the proximal hemisphere (32) has first anchoring means (34), and/or wherein the distal hemisphere (35) has second anchoring means (36).

11. System for inserting an occluder (12) into a patient and for arranging the occluder (12) on the left atrial appendage (10) of the patient, the system comprising an insertion catheter (104) and an occluder (12) comprising biological tissue (40) according to any of the preceding claims.

12. Method for providing a system according to claim 11, wherein biological tissue (40) is first stabilized by means of a reticulation method and, in order to make it durable, is either wetted with a liquid medium, or dried.

13. Method according to claim 12, wherein the liquid medium comprises glutaraldehyde and/or alcohol and the occluder (12) comprising the biological tissue (40) is arranged on the insertion catheter (104) immediately before it is inserted into the patient.

14. Method according to claim 12, wherein the biological tissue (40) is vacuum-dried or air-dried, and wherein the occluder (12) comprising the biological tissue (40) is introduced into the insertion catheter (104) and, having been made durable, is made available.

## Revendications

1. Dispositif d'occlusion (12) pour fermer l'auricula cordis sinistra (10) d'un patient, comprenant un cadre (14) auto-expansible, **caractérisé en ce que** le dispositif d'occlusion (12) présente à l'état expansé un contour extérieur sensiblement sphérique, dans lequel un tissu biologique (40) est disposé sur le cadre (14), qui recouvre au moins par sections l'hémisphère proximal (32) du côté extérieur du cadre (14), que le tissu (40) est réalisé pour fermer de manière étanche aux fluides l'auricula cordis sinistra (10) à l'état disposé du dispositif d'occlusion (12), et que le tissu biologique (40) est stabilisé par traitement au moyen d'une méthode de réticulation et soit mouillé avec un milieu liquide, soit séché pour la conservation.

2. Dispositif d'occlusion (12) selon la revendication 1, **caractérisé en ce que** le tissu biologique (40) est réalisé sous la forme d'une membrane péricardique.

3. Dispositif d'occlusion (12) selon l'une quelconque des revendications précédentes, **caractérisé en ce que** la méthode de réticulation comprend le procédé de réticulation utilisant notamment du glutaraldéhyde et/ou de l'alcool.

4. Dispositif d'occlusion (12) selon l'une quelconque des revendications 1 à 3, **caractérisé en ce que** le milieu liquide avec lequel le tissu biologique (40) est mouillé est du glutaraldéhyde et/ou de l'alcool.

5. Dispositif d'occlusion (12) selon l'une quelconque des revendications 1 à 3, **caractérisé en ce que** le tissu biologique (40) est d'abord mouillé avec du glycérol et/ou avec un alcool mono ou trivalent, puis séché.

6. Dispositif d'occlusion (12) selon la revendication 5, **caractérisé en ce que** le tissu biologique (40) est séché sous vide ou séché à l'air.

7. Dispositif d'occlusion (12) selon la revendication 6, **caractérisé en ce que** le tissu biologique (40) séché est stérilisé avec de l'oxyde d'éthylène.

8. Dispositif d'occlusion (12) selon l'une quelconque des revendications précédentes, **caractérisé en ce que** le dispositif d'occlusion (12) comprend une extrémité proximale (142) et une extrémité distale (140), et que, dans une position d'introduction, le dispositif d'occlusion (12) présente par rapport à la forme auto-expansible un contour extérieur allongé dans lequel l'extrémité proximale (142) est déplacée dans la direction proximale et l'extrémité distale (140) dans la direction distale et le dispositif d'occlusion (12) est étiré.

9. Dispositif d'occlusion (12) selon l'une quelconque des revendications précédentes, dans lequel le cadre (14) comprend à son extrémité proximale une section d'extrémité tubulaire (16) et/ou dans lequel le cadre (12) comprend à son extrémité distale une section d'extrémité en forme de pot (18).

10. Dispositif d'occlusion (12) selon l'une quelconque des revendications précédentes, dans lequel l'hémisphère proximal (32) comprend des premiers moyens d'ancrage (34), et/ou dans lequel l'hémisphère distal (35) du dispositif d'occlusion (12) comprend des deuxièmes moyens d'ancrage (36).

11. Système pour l'introduction d'un dispositif d'occlusion (12) dans un patient et pour la disposition du dispositif d'occlusion (12) sur l'auricula cordis sinistra (10) du patient, le système comprenant un cathéter d'introduction (104) et un dispositif d'occlusion (12) avec du tissu biologique (40) selon l'une quelconque des revendications précédentes.

12. Procédé pour fournir un système selon la revendication 11, dans lequel un tissu biologique (40) est d'abord stabilisé par une méthode de réticulation et, pour la conservation, est soit mouillé avec un milieu liquide, soit séché.

13. Procédé selon la revendication 12, dans lequel le milieu liquide comprend du glutaraldéhyde et/ou de l'alcool et le dispositif d'occlusion (12) avec le tissu biologique (40) est disposé sur le cathéter d'introduction (104) immédiatement avant son introduction dans le patient.

14. Procédé selon la revendication 12, dans lequel le tissu biologique (40) est séché sous vide ou séché à l'air et dans lequel le dispositif d'occlusion (12) avec le tissu biologique (40) est inséré dans le cathéter d'introduction (104) et mis à disposition de manière conservée.
